# EUROPÄISCHE PATENTSCHRIFT

(11) **EP 1 165 834 B1**
(45) Veröffentlichungstag und Bekanntmachung des Hinweises auf die Patenterteilung: **15.09.2004**
(21) Anmeldenummer: 00909352.7
(22) Anmeldetag: 10.03.2000
(51) Int. Cl.: C12Q 1/44, G01N 33/58

(54) **HIGH-THROUGHPUT-SCREENING-VERFAHREN ZUR BESTIMMUNG VON ENANTIOSELEKTIVITÄT**
HIGH THROUGHPUT SCREENING METHOD FOR DETERMINING ENANTIOSELECTIVITY
PROCEDE DE DEPISTAGE A DEBIT ELEVE POUR LA DETERMINATION DE L'ENANTIO-SELECTIVITE

(30) Priorität: 26.03.1999 DE 19913858
(43) Veröffentlichungstag der Anmeldung: 02.01.2002
(73) Patentinhaber: Studiengesellschaft Kohle mbH, 45470 Mülheim (DE)
(72) Erfinder: REETZ, Manfred, Theodor, D-45470 Mülheim an der Ruhr (DE); BECKER, Michael, Heinrich, D-45470 Mülheim an der Ruhr (DE); STÖCKIGT, Detlef, D-45470 Mülheim an der Ruhr (DE); KLEIN, Heinz-Werner, D-45470 Mülheim an der Ruhr (DE)
(86) Internationale Anmeldenummer: PCT/EP2000/002121
(87) Internationale Veröffentlichungsnummer: WO 2000/058504

(56) Entgegenhaltungen:
- WO-A-99/05288
- DATABASE CHEMICAL ABSTRACTS [Online] AMERICAN CHEMICAL SOCIETY (VIA STN KARLSRUHE); Accession Number 1999:145438 CAPLUS, GUO, J. ET AL: "Measurement of enantiomeric excess by kinetic resolution and mass spectrometry" XP002141731 & "BOOK OF ABSTRACTS, 217TH ACS NATIONAL MEETING, ANAHEIM, CALIF. (21-25 MARCH 1995)" , AMERICAN CHEMICAL SOCIETY , WASHINGTON, D.C.
- REETZ ET AL: "Creation of enantioselective biocatalysts for organic chemistry by in vitro evolution" ANGEWANDTE CHEMIE. INTERNATIONAL EDITION,DE,VERLAG CHEMIE. WEINHEIM, Bd. 36, Nr. 24, 1. Januar 1998 (1998-01-01), Seiten 2830-2832, XP002087468 ISSN: 0570-0833
- JAEGER K -E ET AL: "Microbial lipases form versatile tools for biotechnology" TRENDS IN BIOTECHNOLOGY,NL,ELSEVIER, AMSTERDAM, Bd. 16, Nr. 9, September 1998 (1998-09), Seiten 396-403, XP004173187 ISSN: 0167-7799
- REETZ M T ET AL: "TIME-RESOLVED IR-THERMOGRAPHIC DETECTION AND SCREENING OF ENANTIOSELECTIVITY IN CATALYTIC REACTIONS" ANGEWANDTE CHEMIE. INTERNATIONAL EDITION,DE,VERLAG CHEMIE. WEINHEIM, Bd. 37, Nr. 19, 1998, Seiten 2647-2650, XP000901132 ISSN: 0570-0833
- L E JANES, R J KAZLAUSKAS: "Quick E. A Fast Spectrophotometric Method To Measure the Enantioselectivity of Hydrolases " JOURNAL OF ORGANIC CHEMISTRY, Bd. 62, 1997, Seiten 4560-4561, XP002141728 in der Anmeldung erwähnt
- L E JANES, C LÖWENDAHL, R J KAZLAUSKAS: "Quantitative Screening of Hydrolase Libraries Using pH Indicators: Identifying Active and Enantioselective Hydrolases" CHEMISTRY - A EUROPEAN JOURNAL, Bd. 4, Nr. 11, 1998, Seiten 2324-2331, XP002141729 in der Anmeldung erwähnt
- REETZ, MANFRED T. ET AL: "A method for high-throughput screening of enantioselective catalysts" ANGEW. CHEM., INT. ED. (1999), 38(12), 1758-1761 , XP002141730
- JAEGER KARL-ERICH; REETZ MANFRED T: "Directed evolution of enantioselective enzymes for organic chemistry" CURRENT OPINION IN CHEMICAL BIOLOGY, Bd. 4, Februar 2000 (2000-02), Seiten 68-73, XP000922816
- SO, M. P. ET AL: "Differentiation of enantiomers using matrix-assisted laser desorption/ionization mass spectrometry" RAPID COMMUN. MASS SPECTROM. (2000), 14(8), 692-695 , XP000922962

## Beschreibung

Die vorliegende Erfindung betrifft ein Verfahren zur Bestimmung der Enantioselektivität von kinetischen Racematspaltungen und von asymmetrisch verlaufenden Reaktionen prochiraler Verbindungen, die enantiotope Gruppen tragen, in dem isotopenmarkierte Substrate verwendet werden, so daß mit einem isotopenspezifischen Detektionssystem, z. B. einem ESI-Massenspektrometer, die Reaktionsprodukte quantitativ bestimmt werden können. In Verbindung mit einer automatisierten Probenaufgabe kann das Verfahren zum *High-Throughput-Screening* eingesetzt werden. Die dabei relevanten enantioselektiven Stoffumwandlungen können durch chirale homogene oder heterogene Katalysatoren, Biokatalysatoren oder durch stöchiometrische Mengen optisch aktiver Agenzien induziert werden.

Die Entwicklung effektiver Verfahren zur Generierung umfangreicher Bibliotheken chiraler Chemie-Katalysatoren durch Methoden der kombinatorischen Chemie [a) G. Liu, J. A. Ellman, *J*. *Org. Chem*. **1995,** *60,* 7712-7713; b) K. Burgess, H.-J. Lim, A. M. Porte, G. A. Sulikowski, *Angew*. *Chem*. **1996,** *108,* 192-194; *Angew*. *Chem*., *Int*. *Ed*. *Engl.* **1996,** 35, 220-222; c) B. M. Cole, K. D. Shimizu, C. A. Krueger, J. P. A. Harrity, M. L. Snapper, A. H. Hoveyda, *Angew*. *Chem.* **1996,** *108*, 1776-1779; *Angew*. *Chem*., *Int*. *Ed. Engl.* **1996,** 35, 1668-1671; d) C. Gennari, H. P. Nestler, U. Piarulli, B. Salom, *Liebigs Ann.*/*Recl*. **1997,** 637-647] oder zur Herstellung von Bibliotheken enantioselektiver Biokatalysatoren durch *in vitro*-Evolution [M. T. Reetz, A. Zonta, K. Schimossek, K. Liebeton, K.-E. Jaeger, *Angew. Chem*. **1997,** *109,* 2961-2963; *Angew*. *Chem*., *Int*. *Ed*. *Engl*. **1997,** *36*, 2830-2932] ist Gegenstand der aktuellen Forschung. Entscheidend für den Erfolg dieser neuen Technologien ist das Vorhandensein von effektiven und raschen Methoden zum Durchsuchen und Auffinden der enantioselektivsten Katalysatoren oder Biokatalysatoren aus den jeweiligen Katalysator-Bibliotheken. Während in der kombinatorischen Wirkstoffchemie viele effektive Methoden zum Durchsuchen großer Bibliotheken von biologisch aktiven Verbindungen zur Verfügung stehen [a) F. Balkenhohl, C. von dem Bussche-Hünnefeld, A. Lansky, C. Zechel, *Angew*. *Chem*. **1996,** *108,* 2436-2488; *Angew. Chem*., *Int*. *Ed*. *Engl*. **1996,** *35*, 2288-2337; b) J. S. Früchtel, G. Jung, *Angew. Chem*. **1996,** *108,* 19-46; *Angew. Chem., Int.* *Ed*. *Engl.* **1996**, *35*, 17-42; c) *Chem*. *Rev.* **1997,** *97* (2), 347-510 (Sonderausgabe über kombinatorische Chemie); d) S. R. Wilson, A. W. Czarnick, *Combinatorial Chemistry: Synthesis and Application,* Wiley, New York, **1997**], steht die Entwicklung von Methoden zum *High-Throughput-Screening* von enantioselektiven Katalysatoren, Biokatalysatoren oder optisch aktiven Agenzien erst am Anfang. Die Bestimmung des Enantiomerenüberschusses (engl. enantiomeric excess, *ee*) der Produkte stereoselektiver Stoffumwandlungen erfolgt normalerweise klassisch mit Hilfe der Gas- oder Flüssigkeitschromatographie an chiralen stationären Phasen [G. Schomburg, *Gaschromatographie: Grundlagen, Praxis, Kapillartechnik*, 2. Aufl., VCH, Weinheim, **1987;** K. K. Unger, *Packings and stationary phases in chromatographic techniques,* Series Chromatographic science; v. 47, Marcel Dekker, New York, **1990**]. Obwohl hierbei genaue *ee*-Werte ermittelt werden können, haben solche herkömmlichen Verfahren den Nachteil, dass nur eine begrenzte Zahl von Proben pro Zeiteinheit untersucht werden kann, denn die Analysenzeiten hängen von den jeweiligen Retentionszeiten ab.

Um Analysenprobleme dieser Art zu lösen, wurden kürzlich die ersten Vorschläge gemacht. So wurde z. B. im Rahmen einer Untersuchung zur *in vitro*-Evolution von enantioselektiven Lipasen ein relativ grobes Testverfahren entwickelt, wonach der Verlauf enantioselektiver Hydrolysen von chiralen Carbonsäureestern ermittelt werden kann [M. T. Reetz, A. Zonta, K. Schimossek, K. Liebeton, K.-E. Jaeger, *Angew. Chem*. **1997**, *109,* 2961-2963; *Angew. Chem., Int*. *Ed*. *Engl*. **1997**, *36*, 2830-2932]. Dabei werden die von Lipase-Mutanten katalysierten Hydrolysen von (*R*)- und (*S*)-konfigurierten Carbonsäure-*p*-nitrophenolestern auf Mikrotiterplatten spektrophotometrisch zeitlich verfolgt, wodurch die enantioselektivsten Mutanten rasch identifiziert werden können. Abgesehen davon, dass keine exakten *ee*-Werte zugänglich sind, ist dieses Verfahren auf die Stoffklasse der chiralen Carbonsäuren beschränkt. Gleiches gilt für eine verwandtes Testverfahren [L. E. Janes, R. J. Kazlauskas, *J. Org. Chem*. **1997**, *62*, 4560-4561]. Diese Einschränkung gilt auch für verwandte Verfahren, die auf Farbänderung von pH-Indikatoren während einer Esterhydrolyse beruhen [L. E. Janes, A. C. Löwendahl, R. J. Kazlauskas, *Chem*. *- Eur. J.* **1998**, *4*, 2324-2331]. Ein völlig anderer Ansatz zur Identifizierung chiraler Katalysatoren bezieht sich auf IR-Thermographie [M. T. Reetz, M. H. Becker, K. M. Kühling, A. Holzwarth, *Angew*. *Chem*. **1998**, *110*, 2792-2795; *Angew. Chem., Int*. *Ed*. **1998**, *37*, 2547-2650]. Allerdings steht die Weiterentwicklung dieser Methode unter Ermöglichung einer quantitativen Analyse enantioselektiver Reaktionen noch aus.

Die vorliegende Erfindung behebt diese Unzulänglichkeiten dadurch, daß für kinetische Racematspaltungen oder auch für stereoselektive Reaktionen mit enantiotope Gruppen enthaltenden prochiralen Substraten teilweise oder vollständig isotopenrnarkierte Substrate oder Substrate mit einer von der natürlichen Verteilung abweichenden Isotopenverteilung eingesetzt werden. Dies erlaubt den Einsatz eines isotopenspezifischen Detektionssystems, z. B. einer massenspektrometrischen lonisicrungsmethode, zur quantitativen Bestimmung des Umsatzes oder der relativen Verhältnisse der pseudo-Enantiomeren oder der Enantiomerenüberschüsse. Als massenspektrometrische Ionisierungsmethode können APCI (atmospheric pressure chemical ionization), CI (chemical ionization), EI (electron ionization), ESI (electrospray ionization), FAB (fast atom bombardment), FD (field desorption), FI (field ionization), LILBID (laser induced liquid beam ionization desorption), LSIMS (liquid secondary ion mass spectrometry), MALDI (matrix-assisted laser desorption/ionization), PB (particle beam), PD (plasma desorption), SIMS (secondary ion mass spectrometry), oder TSP (thermospray) oder eine Kombination solcher Ionisierungsmethoden angewandt werden.

Im Vergleich zu den bisherigen Ansätzen bietet die vorliegende Erfindung folgende Vorteile:
1) Exakte Bestimmung der *ee*-Werte von kinetischen Racematspaltungen und von asymmetrisch verlaufenden Stoffumwandlungen prochiraler Verbindungen, die enantiotope Gruppen tragen, wobei keine Einschränkungen hinsichtlich der Stoffklasse oder des Reaktionstyps gemacht werden.
2) Exakte Bestimmung des Umsatzes der unter 1) erwähnten Reaktionen.
3) Der rasche bzw. der Hochdurchsatztest der unter 1) und 2) erwähnten Daten, wobei speziell mindestens 1000 Bestimmungen pro Tag möglich sind.

Die in der vorliegenden Erfindung verwendeten Detektionssysteme sind Massenspektrometer, insbesondere solche mit Elektrospray Ionisierung (ESI) [J. B. Fenn, M. Mann, C. K. Meng, S. F. Wong, C. M. Whitehouse, *Science (Washington, D. C.)* **1989,** *246*, 64-71] oder Matrix-unterstützte Laserdesorption/ionisierung (MALDI) [a) K. Tanaka, H. Waki, Y. Ido, S. Akita, Y. Yoshida, T. Yoshida, *Rapid Commun. Mass Spectrom*. **1988,** *2*, 151-153; b) M. Karas, F. Hillenkamp, *Anal. Chem.* **1988,** *60*, 2299-2301]. Das erfindungsgemäße Verfahren eignet sich in Verbindung mit einer automatisierten Probenaufgabe (Verwendung von einem oder mehreren Probenaufgaberobotem und Mikrotiterplatten), evtl. auch unter Einsatz mehrerer Spektrometer, als High-Throughput-Screening Methode.

Das Verfahren kann verwendet werden um chirale Katalysatoren oder chirale Agenzien für asymmetrisch verlaufende Reaktionen aufzufinden bzw. zu optimieren. Hierzu zählen
a) chirale Katalysatoren oder chirale Agenzien für die kinetische Racematspaltung von Alkoholen, Carbonsäuren, Carbonsäureestern, Aminen, Amiden, Olefinen, Alkinen, Phosphinen, Phosphoniten, Phosphiten, Phosphaten, Halogeniden, Oxiranen, Thiolen, Sulfiden, Sulfonen, Sulfoxiden, Sulfonamiden sowie deren Derivate;
b) chirale Katalysatoren (z. B. chirale homogene oder chiral-modifizierte heterogene Katalysatoren, chirale Metallkomplexe) oder chirale Agenzien für die stereoselektive Umsetzung von prochiralen Verbindungen, deren enantiotope Gruppen eine oder mehrere funktionelle Gruppen aus den Substanzklassen der Alkohole, Carbonsäuren, Carbonsäureester, Amine, Amide, Olefine. Alkine, Phosphine, Phosphonite, Phosphite, Phosphate, Halogenide, Oxirane, Thiole, Sulfide, Sulfone, Sulfoxide oder Sulfonamide oder deren Derivate umfassen;
c) Biokatalysatoren, z. B. Enzyme, Antikörper, Proteine, Hormone, Phagen, Ribozyme, Peptide oder sonstige Biopolymere, für die kinetische Racematspaltung von Alkoholen, Carbonsäuren, Carbonsäureestern, Aminen, Amiden, Olefinen, Alkinen, Phosphinen, Phosphoniten, Phosphiten, Phosphaten, Halogeniden, Oxiranen, Thiolen, Sulfiden, Sulfonen, Sulfoxiden, Sulfonamiden sowie deren Derivate und für die die stereoselektive Umsetzung von prochiralen Verbindungen, deren enantiotope Gruppen eine oder mehrere funktionelle Gruppen aus den Substanzklassen der Alkohole, Carbonsäuren, Carbonsäureester, Amine, Amide, Olefine, Alkine, Phosphine, Phosphonite, Phosphite, Phosphate, Halogenide, Oxirane, Thiole, Sulfide, Sulfone, Sulfoxide oder Sulfonamide oder deren Derivate.

Das zugrunde liegende Prinzip der Erfindung basiert auf der Verwendung von isotopenmarkierten Substraten in Form von *pseudo*-Enantiomeren oder *pseudo*prochiralen Verbindungen, wie in Schema 1 dargestellt.

Wird in einem herkömmlichen Racemat die eine enantiomere Form isotopenmarkiert, so nennt män solche Verbindungen *pseudo*-Enantiomere. Markiert man eine enantiotope Gruppe eines prochiralen Substrats mit Isotopen, so nennt man die Verbindung *pseudo*-prochiral, so z. B. *pseudo-meso.* Je nachdem welche Typen von enantioselektiven Umwandlungen im Rahmen der Erfindung untersucht bzw. getestet werden sollen, sind unterschiedliche Situationen relevant, so z. B. Fall a, Fall b, Fall c und Fall d in Schema 1. Bei kinetischen Racematspaltungen von beliebigen chiralen Verbindungen werden 1 und 2, die sich in der absoluten Konfiguration und in der Isotopenmarkierung in den funktionellen Gruppen FG* unterscheiden, in enantiomerenreiner Form hergestellt und im Verhältnis von 1 : 1 gemischt, so dass ein Racemat simuliert wird (Schema 1a). Nach einer enantioselektiven Umwandlung, bei der die chemische Reaktion an der funktionellen Gruppe eintritt (im Idealfall einer kinetischen Antipodenspaltung bis zu einem Umsatz von 50 %), entstehen echte Enantiomere **3** und **4** neben nichtmarkierten und markierten achiralen Nebenprodukten **5** bzw. **6.**
Die Verhältnisse der Intensitäten von **1/2** und **5/6** in den Massenspektren (*m*/*z* Intensitäten der Quasi-Molekülionen) erlauben die quantitative Ermittlung der Enantioselektivität (ee-Werte) und des Umsatzes. Gegebenenfalls kann im Rahmen der Erfindung auch ein innerer Standard verwendet werden. Von Fall zu Fall kann es vorteilhaft sein, die Isotopenmarkierung nicht in der funktionellen Gruppe, sondern im Rest R² des Substrats vorzunehmen, wie in Schema 1b skizziert. In diesem Fall entsteht ein neues Paar von *pseudo*-Enantiomeren **3/8** (Schema 1b), wobei Enantioselektivität und Umsatz durch Messung der m/z-Intensitäten der Quasi-Molekülionen von **1/7** und **3/8** erfindungsgemäß ermittelt werden. Somit sind in beiden Fällen auch die sogenannten Selektivitätsfaktoren (s- oder E-Werte [H. B. Kagan, J. C. Fiaud in *Top. Stereochem.,* Vol. 18 (Hrsg.: E. L. Eliel, S. H. Wilen), Wiley, New York, **1988**, S. 249-330] automatisch zugänglich.

Im Falle von prochiralen Substraten mit enantiotopen Gruppen ist im Rahmen des erfindungsgemäßen *Screening*systems die Synthese einer einzigen *pseudo*-prochiralen Verbindung erforderlich. Ist das relevante Substrat eine *meso*-Verbindung, so wird zunächst eine entsprechende *pseudo-meso*-Verbindung **9** hergestellt, denn die zu untersuchende stereo-differenzierende Reaktion ergibt eine Mischung von zwei MS-detektierbaren *pseudo*-Enantiomeren **10** und **11** (Schema 1c). Erfindungsgemäß gilt die analoge Vorgehensweise für andere prochirale Substrate mit enantiotopen Gruppen, so z. B. die Verwendung von *pseudo*-prochiralen Substraten des Typs **12** (Schema 1d).

Die FG-Einheiten in Schema 1 können die unterschiedlichsten funktionellen Gruppen der organischen Chemie sein. Typische Vertreter sind Acyloxy-Reste (-OC(O)R), Thioacyloxy-Reste (-SC(O)R), Amido-Reste (-NHC(O)R), Carboxyl-Reste (-CO₂R) im Falle von Spaltungsreaktionen wie z. B. Hydrolysen, ferner Hydroxy-Reste (-OH), Thiol-Reste (-SH), Amino-Reste (-NH₂ oder -NHR) oder Carboxy-Reste (-CO₂H) im Falle von verknüpfenden Reaktionen wie Acylierungen oder Veresterungen.

Schema 1 dient nur zur Illustration bzw. Beschreibung der erfindungsgemäßen Methode und schränkt diese in keiner Weise ein. Vielmehr sind die unterschiedlichsten Substanzklassen und Reaktionstypen möglich, sofern die Substrate entweder chiral sind wie naturgemäß bei einer kinetischen Racematspaltung oder sie sind prochiral und enthalten enantiotope Gruppen.

Um einen hohen Probendurchsatz zu gewährleisten, sieht die Erfindung einen Geräteaufbau vor, wie er beispielhaft in Schema 2 skizzierte ist. Mit dieser Kombination von kommerziell erhältlichen Geräten bzw. Apparateteilen ist es möglich, mindestens 1000 *ee*-Bestimmungen pro Tag mit einer Genauigkeit von ± 5 % durchzuführen.

### Beispiel 1. Kinetische Racematspaltung von 1-Phenylethylacetat

Die hydrolytische kinetische Racematspaltung von 1-Phenylethylacetat, katalysiert durch z. B. Enzyme wie Lipasen (Wildtyp oder Mutanten) wird mit der in Schema 2 beschriebenen Konfiguration im Rahmen eines *High-Throughput-Screenings* verfolgt, d. h. es werden *ee*- und Umsatzbestimmungen durchgeführt.

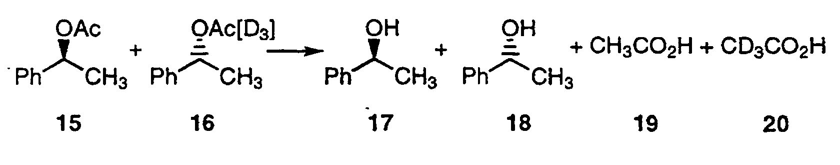

### Synthese von (1S)-1-Phenylethylacetat (15):

In einem 25 ml-Stickstoffkolben werden unter Luft- und Feuchtigkeitsausschluss 8.28 mmol (1.01 g, 1.00 ml) (*S*)-1-Phenylethanol (Fluka, 99 % *ee*), 12.4 mmol (1.50 Äq., 983 mg, 1.00 ml) Pyridin und 15 ml Dichlormethan vorgelegt und auf 0°C (Eisbad) gekühlt. Unter Rühren werden innerhalb von 10 min 10.8 mmol (1.30 Äq., 1.10 g, 1.02 ml) Acetanhydrid zugetropft. In einem Zeitraum von 12 h wird auf Raumtemperatur erwärmt und sukzessive mit je zweimal 20 ml 1 M Salzsäurelösung, gesättigter Natriumhydrogencarbonatlösung und gesättigter Natriumchloridlösung extrahiert. Die organische Phase wird über Magnesiumsulfat getrocknet, vom Trockenmittel durch Filtration getrennt und am Rotationsverdampfer vom Lösungsmittel befreit. Das Rohprodukt wurde auf Kieselgel aufgetragen und chromatographisch gereinigt (Laufmittel: Hexan : Essigsäureethylester = 9 : 1). Die Produktfraktionen werden vereinigt und am Rotationsverdampfer vom Lösungsmittel befreit. Nach Trocknen im Ölpumpenvakuum verbleibt eine klare Flüssigkeit (1.25 g, 7.62 mmol, 92 %). Analytik: ¹H-NMR (CDCl₃, 200 MHz): δ = 1.52 (d) ³*J* = 6.6 Hz [3H], 2.06 (s) [3H], 5.94 (q) ³*J* = 6.6 Hz [1H], 7.24-7.38 (m) [5H]; ¹³C-{1H}-NMR (CDCl₃, 50 MHz): δ = 21.4 (s), 22.2 (s). 72.3 (s), 126.1 (s), 127.9 (s), 128.5 (s), 141.7 (s), 170.3 (s); MS (EI, 70 eV, pos. Ionen): *m*/*z* (% rel. Int.): 164 (25) [M⁺], 122 (77), 107 (36), 105 (69), 104 (100). 103 (24), 79 (27), 78 (27), 77 (43), 51 (24), 43 (90); EA: C: 72.95 % (ber. 73.15 %), H: 7.28 % (ber. 7.37 %); GC (Hewlett Packard 5890, 25 m *fused silica*, 0.25 innerer Durchmesser, 2,6-Dimethyl-3-pentyl-β-CD (95 % Methyl-/ 5 % Phenylpolysiloxan), FID, 80 °C, 4 °C min⁻¹, 120 °C 0.2 min iso, 0.63 bar Wasserstoff): 99.8 % *ee*.

### Synthese von (1R)-1-Phenylethyl-trideuteroacetat (16):

In einem 25 ml-Stickstoffkolben werden unter Luft- und Feuchtigkeitsausschluss 8.28 mmol (1.01 g, 1.00 ml) (*R*)-1-Phenylethanol (Fluka, 99 % *ee*), 12.4 mmol (1.50 Äq., 983 mg, 1.00 ml) Pyridin und 15 ml Dichlormethan vorgelegt und auf 0 °C (Eisbad) gekühlt. Unter Rühren werden innerhalb von 10 min 10.8 mmol (1.3 Äq., 1.16 g, 1.02 ml) *d*₆-Acetanhydrid (99 Atom% D, Aldrich) zugetropft. Innerhalb von 12 h wird auf Raumtemperatur erwärmt und sukzessive mit je zweimal 20 ml 1 M Salzsäurelösung, gesättigter Natriumhydrogencarbonatlösung und gesättigter Natriumchloridlösung extrahiert. Die organische Phase wird über Magnesiumsulfat getrocknet, vom Trockenmittel durch Filtration getrennt und am Rotationsverdampfer vom Lösungsmittel befreit. Das Rohprodukt wurde auf Kieselgel aufgetragen und chromatographisch gereinigt (Laufmittel: Hexan : Essigsäureethylester = 9 : 1). Die Produktfraktionen werden vereinigt und am Rotationsverdampfer vom Lösungsmittel befreit. Nach Trocknen im Ölpumpenvakuum verbleibt eine klare Flüssigkeit (1.28 g, 7.65 mmol, 92 %). Analytik: ¹H-NMR (CDCl₃, 200 MHz): δ = 1.53 (d) ³*J* = 6.6 Hz [3H], 5.88 (q) ³*J* = 6.6 Hz [1H], 7.24-7.36 (m) [5H]; ¹³C-{1H}-NMR (CDCl₃, 50 MHz): δ = 22.2 (s), 72.3 (s), 126.1 (s), 127.7 (s), 128.5 (s), 141.7 (s), 170.3 (s); MS (EI, 70 eV, pos. Ionen): *m*/*z* (% rel. Int.): 167 (24) [M⁺], 123 (77), 108 (39), 105 (67), 104 (100), 103 (25), 78 (27), 77 (35), 51 (19), 46 (65), 43 (14); EA: C: 71.77 % (ber. 71.83 %), H+D: 6.98 % (ber. 7.23 %); GC (Hewlett Packard 5890, 25 m *fused silica*, 0.25 innerer Durchmesser, 2,6-Dimethyl-3-pentyl-β-CD (95 % Methyl-/ 5 % Phenylpolysiloxan), FID, 80 °C, 4 °C min⁻¹, 120 °C 0.2 min iso, 0.63 bar Wasserstoff): 99.8 % *ee.*

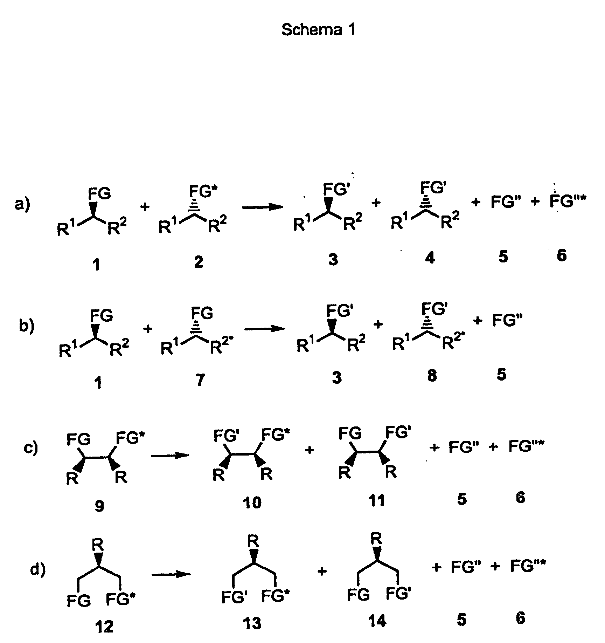

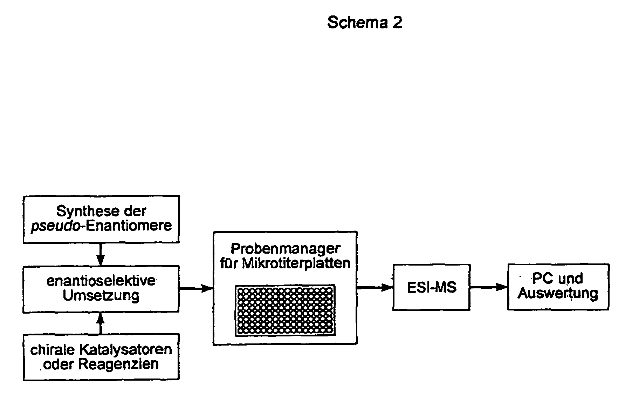

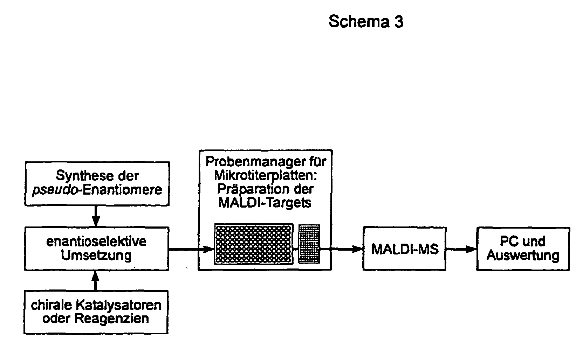

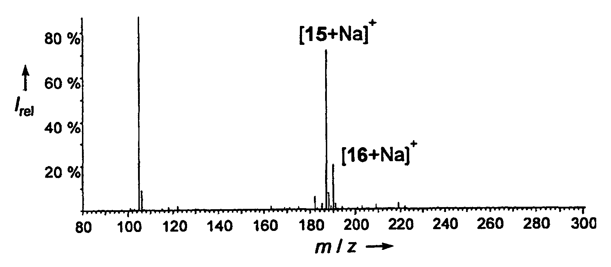

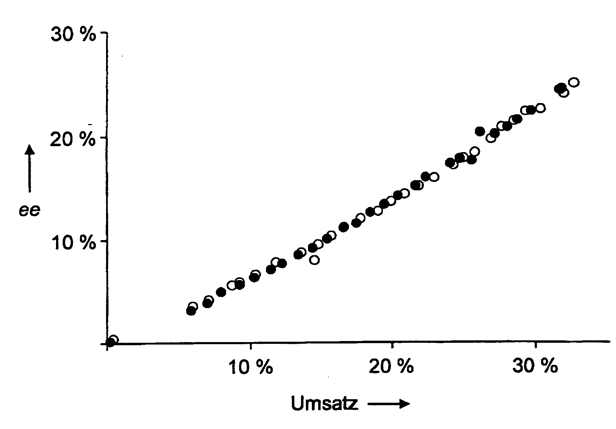

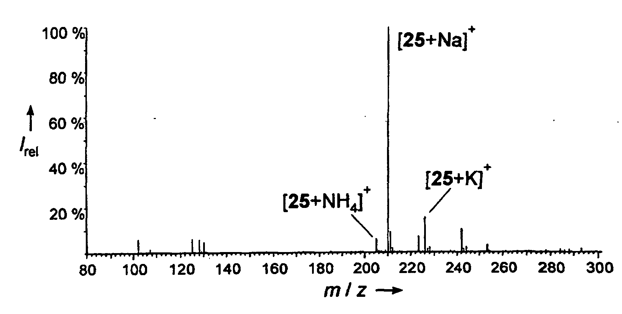

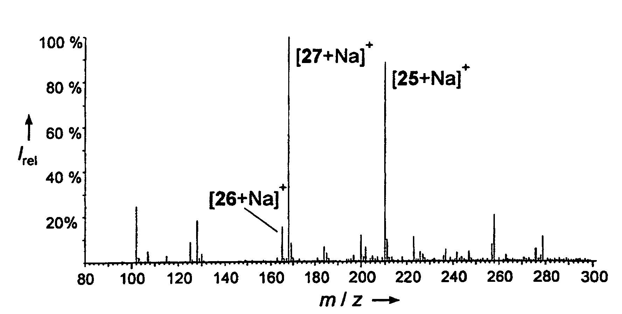

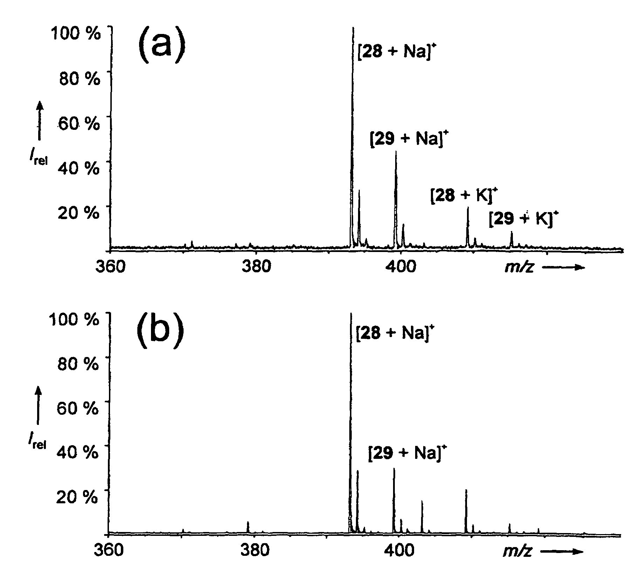

In Vorversuchen wurden die *pseudo*-Enantiomere **15** und **16** in verschiedenen Verhältnissen gemischt. Die dabei resultierenden Mischungen wurden zunächst mit Hilfe der herkömmlichen Gaschromatographie an chiraler stationärer Phase untersucht, um die *pseudo-ee*-Werte zu ermitteln. Die gleichen Proben wurden dann mit ESI-MS untersucht. Ein typisches ESI-Massenspektrum ist in Abb. 1 gezeigt. Der Vergleich der zwei Datensätze zeigt Übereinstimmung im Rahmen von ± 5 % (Tabelle 1).

**Tabelle 1**

| Probe | *ee* (GC) | *ee* (ESI-MS) |
|---|---|---|
| 1 | 100 | 100 |
| 2 | 91 | 90 |
| 3 | 81 | 79 |
| 4 | 74 | 73 |
| 5 | 60 | 57 |
| 6 | 56 | 54 |
| 7 | 48 | 48 |
| 8 | 28 | 27 |
| 9 | 10 | 10 |
| 10 | 23 | 20 |
| 11 | 40 | 38 |
| 12 | 45 | 43 |
| 13 | 55 | 53 |
| 14 | 65 | 60 |
| 15 | 75 | 74 |
| 16 | 95 | 93 |
| 17 | 100 | 100 |

Sodann wurden 1:1-Gemische der *pseudo*-Enantiomere **15/16** mit unterschiedlichen Lipasen (Wildtyp und Mutanten, z. B. aus *P. aeruginosa*) auf Mikrotiterplatten hydrolysiert und mit dem in Schema 2 beschriebenen Screeningsystem auf Enantioselektivität und Umsatz untersucht. Dabei ließen sich etwa 1000 exakte *ee*-Bestimmungen pro Tag durchführen. Im einzelnen wurden 10 µL einer 1 mM 1 : 1-Mischung von **15** und **16** in CH₃OH wurden in das Rheodyne®-Ventil eines ESI-MS-Systems (ESI-MS-Bedingungen: HP 5989B MS Engine Quadropol Massenspektrometer ausgerüstet mit einer HP 59987A API Elektrosprayquelle II mit Hexapol lonenführung (Analytica of Branford) und ChemStation®; Datenaufnahme: Scan-Spektren im positiv Ionenmodus; *m*/*z* 90-300; in *m*/*z* 0.1 Schritten, Einheitsauflösung, Gauss' Massenfilter *m*/*z* 0.3 min; Gauss' Zeitfilter 0.05 min; API-Quellen-Bedingungen: Potentialdifferenz zwischen der *Spray*-Nadel und erster Elekrode: -5250 V, Druck des N₂-*Nebulizer*-Gases: 80 psi, Fluß des N₂-Trocknungsgases ca. 9 L min⁻¹ (150°C), Lösungsmittelfluss 0.06 mL min⁻¹, CH₃OH : H₂O = 8 : 2). Es wurden ca. 20-30 ESI-Massenspektren summiert und die Verhältnisse von **15** und **16** wurden durch die absoluten Intensitäten der Signale der entsprechenden Natriumaddukte ([**15**+Na]⁺ und [**16**+Na]⁺, Abb. 1) bestimmt. Die Verhältnisse der Signale (und somit auch die *ee*-Werte der synthetischen Mischungen von **15** und **16**) wurden durch ein Makro automatisch aus der *m*/*z*-Intensitätsliste der einzelnen Messungen in eine Excel®-Tabelle übertragen.

### Beispiel 2. Kinetische Racematspaltung von 2-Phenylpropionsäure

In diesem Beispiel wird die enantioselektive Veresterung von 2-Phenylpropionsäure im Rahmen einer Lipase-katalysierten kinetischen Racematspaltung beschrieben. Dazu wurden zunächst die *pseudo*-Enantiomere **21/22** synthetisiert.

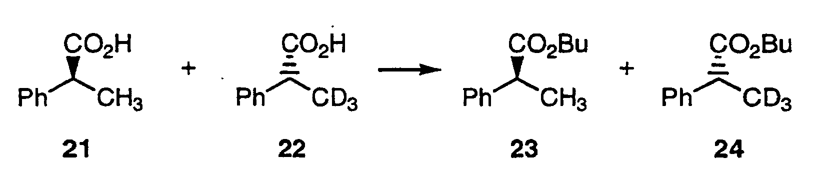

### Synthese von 22:

### Synthese von (4R)-Benzyl-3-N-benzyloxy-2-oxazolidin-2-on:

In einem 250 ml-Stickstoffkolben werden unter Luft- und Feuchtigkeitsausschluss 24.0 mmol (4.52 g) (4*R*)-Benzyloxazolidin-2-on (Fluka) in 80 ml Tetrahydrofuran gelöst und auf -78 °C (Trockeneisbad) gekühlt. Unter Rühren werden innerhalb von 15 min 24.0 mmol (15.0 ml einer 1.6 M Lösung) *n*-Butyllithium in Hexan zugetropft. Nach 90 min wird bei -78 °C eine Lösung von frisch destilliertem Phenylessigsäurechlorid (24.0 mmol, 3.71 g, 3.18 ml) und 10 ml Tetrahydrofuran innerhalb von 10 min zugetropft. Nach beendetem Zutropfen wird das Trockeneisbad durch ein Eisbad ersetzt. Nach Rühren bei 0 °C für 1 h wird die Reaktion durch Zugabe von 25 ml einer gesättigten Ammoniumchlorid-Lösung beendet, und es wird eine weitere Stunde bei 0 °C gerührt. Das Reaktionsgemisch wird im Anschluß in einen 250 ml-Kolben überführt, und das organische Lösungsmittel am Rotationsverdampfer entfernt. Der Rückstand wird dreimal mit 60 ml Dietyhlether, zweimal mit je 70 ml 1 M Natronlauge, 1 M Salzsäure und gesättigter Natriumchloridlösung extrahiert. Die vereinigten organischen Phasen werden über Magnesiumsulfat getrocknet, vom Trockenmittel durch Filtration getrennt und am Rotationsverdampfer vom Lösungsmittel befreit. Das Rohprodukt wurde auf Kieselgel aufgetragen und chromatographisch gereinigt (Laufmittel: Hexan : Essigsäureethylester = 3 : 1). Die Produktfraktionen werden vereinigt und am Rotationverdampfer vom Lösungsmittel befreit. Nach Trocknen im Ölpumpenvakuum verbleibt eine klare Flüssigkeit (5.88 g, 19.9 mmol, 83 %). Analytik: ¹H-NMR (CDCl₃, 300 MHz): δ = 2.67 (dd), ³*J* = 9.5 Hz, ³*J* = 13.4 Hz [1H], 3.27 (dd) ³*J* = 3.3 Hz, ²*J* = 13.4 Hz [1H], 4.14-4.23 (m) [1H], 4.26 (d) ²*J* = 15.7 Hz [1H], 4.35 (d) ²*J* = 15.7 Hz [1H), 4.65-4.70 (m) [1H], 7.25-7.36 (m) [10H]; ¹³C-{1H}-NMR (CDCl₃, 75 MHz): δ = 37.7 (s), 41.6 (s), 55.3 (s), 66.1 (s), 127.26 (s), 127.34 (s), 128.3 (s), 128.6 (s), 128.9 (s), 129.4 (s), 129.8 (s), 130.0 (s), 133.5 (s), 135.1 (s), 153.4 (s), 171.2 (s); MS (EI, 70 eV, pos. Ionen): *m*/*z* (% rel. Int.): 295 (44) [M⁺], 178 (6), 119 (16), 118 (100), 117 (9), 91 (88), 90 (11), 65 (12); EA: C: 73.31 % (ber. 73.20 %), H: 5.72 % (ber. 5.80 %), N: 4.68 % (ber. 4.74 %).

### Synthese von (4R)-Benzyl-3N-((2R)-3,3,3-trideutero-2-phenylpropionyl)-oxazolidin-2-on:

In einem 250 ml-Stickstoffkolben werden unter Luft- und Feuchtigkeitsausschluss 18.0 mmol (3.29 g) Natriumhexamethyldisilazid in ca. 50 ml Tetrahydrofuran gelöst und auf -78 °C (Trockeneisbad) gekühlt. Unter Rühren wird innerhalb von 30 min eine Lösung von 18.0 mmol (5.30 g) (4R)-Benzyl-3N-benzyloxy-2-oxazolidin-2-on in 25 ml Tetrahydrofuran zugetropft. Nach 90 min Rühren bei -78 °C wird eine Lösung aus 90.0 mmol (13.0 g, 5.60 ml) *d*₃-Methyliodid (99 Atom-% D, Aldrich) und 20 ml Tetrahydrofuran innerhalb von 30 min zugetropft. Es wird bei -78 °C für weitere 12 h gerührt und anschließend die Reaktion durch Zugabe von 30 ml einer gesättigten Ammoniumchlorid-Lösung beendet. Nach Erwärmen auf Raumtemperatur wird das Reaktionsgemisch in einen 250 ml-Kolben überführt und das organische Lösungsmittel am Rotationsverdampfer entfernt. Der Rückstand wird dreimal mit 60 ml Dietyhlether extrahiert. Die vereinigten etherischen Phasen werden zweimal mit je 50 ml 10%iger Salzsäure, gesättigter Natriumhydrogencarbonatlösung und gesättigter Natriumchloridlösung extrahiert. Die vereinigten organischen Phasen werden über Magnesiumsulfat getrocknet, vom Trockenmittel durch Filtration getrennt und am Rotationsverdampfer vom Lösungsmittel befreit. Das Rohprodukt wird aus Ether/Essigsäureethylester umkristallisiert. Nach Filtration und Trocknen im Ölpumpenvakuum wird ein farbloser Feststoff erhalten (4.67 g, 14.9 mmol, 83 %). Analytik: ¹H-NMR (CDCl₃, 300 MHz): δ = 2.80 (dd) ³*J* = 9.8 Hz, ²*J* = 13.3 Hz [1H], 3.35 (dd) ³*J* = 3.2 Hz, ²*J* = 13.3 Hz [1H], 4.02-4.13 (m) [2H], 4.55-4.62 (m) [2H], 5.10 (s) [1H], 7.21-7.38 (m) [10H]; ¹³C-{1H}-NMR (CDCl₃, 75 MHz): δ = 37.9 (s), 42.9 (s), 55.8 (s), 65.9 (s), 127.3 (s), 127.4 (s), 128.1 (s), 128.7 (s), 129.0 (s), 129.4 (s), 135.3 (s), 140.2 (s), 152.9 (s), 174.6; MS (EI, 70 eV, pos. Ionen): *m*/*z* (% rel. Int.): 312 (34) [M⁺], 135 (100), 108 (72), 107 (18), 91 (14); EA: C: 73.16 % (ber. 73.05 %), H+D: 6.00 % (ber. 6.13 %), N: 4.41 % (ber. 4.48 %).

### Synthese von (2R)-3,3,3-trideutero-2-phenylpropionsäure (22):

In einem 250 ml-Stickstoffkolben werden 11.6 mmol (3.64 g) (4*R*)-Benzyl-3*N*-((2*R*)-3,3,3-trideutero-2-phenylpropionyl)-oxazolidin-2-on in ca. 100 ml Tetrahydrofuran gelöst und die erhaltene Lösung auf 0 °C gekühlt. Unter Rühren wird eine Lösung von 23.3 mmol (978 mg) Lithiumhydroxid-Monohydrat in 18.0 ml einer 30%igen wäßrigen Wasserstoffperoxidlösung unter Rühren zugegeben. Anschließend wird bei 0 °C für 18 h gerührt. Die Reaktion wird bei 0 °C durch tropfenweise Zugabe von 20 ml einer gesättigten Natriumsulfitlösung beendet, das Reaktionsgemisch in einen 250 ml-Kolben überführt und das organische Lösungsmittel am Rotationsverdampfer entfernt. Der Rückstand wird dreimal mit je 70 ml Dichlormethan extrahiert. Die wäßrige Phase wird mit 10%iger Salzsäure auf pH 5 angesäuert und anschließend dreimal mit je 70 ml Diethylether extrahiert. Die vereinigten etherischen Phasen werden über Magnesiumsulfat getrocknet, vom Trockenmittel durch Filtration abgetrennt und das Lösungsmittel am Rotationsverdampfer entfernt. Nach Trocknung im Ölpumpenvakuum verbleibt eine farblose Flüssigkeit (8.60 mmol, 1.32 g, 74 %). Analytik: ¹H-NMR (CDCl₃, 300 MHz): δ = 3.72 (s) [1H], 7.24-7.35 (m) [5H], 11.5 (s) [1H]; ¹³C-{1H}-NMR (CDCl₃, 75 MHz): δ = 45.2 (s), 127.4 (s), 127.6 (s), 128.7 (s), 139.7 (s), 180.8 (s); MS (EI, 70 eV, pos. Ionen): *m*/*z* (% rel. Int.): 153 (24) [M⁺], 108 (100), 91 (8), 82 (5), 81 (8), 80 (5), 79 (10), 78 (8), 77 (6), 43 (11); EA: C: 70.35 % (ber. 73.56 %), H+D: 6.62 % (ber. 6.58 %); HPLC (Varian 5560, stat. Phase: 250 mm Chiracel OD-H, 4.6 mm i. D., mob. Phase: *n*-Heptan : 2-Propanol: Trifluoessigsäure = 98 : 2 : 0.1, 298 K, 1.2 MPa, 0.5 mL min⁻¹, Detektion: UV 254 nm): 99.4 % *ee*.

Zum Ausschluss von möglicherweise störenden sekundären Isotopeneffekten, wurde die Reaktion der *pseudo*-Enantiomeren **21/22** mit der Reaktion des echten Racemats von **21** in einem Vorversuch verglichen. Die Daten in Abbildung 2 zeigen keine wesentlichen Unterschiede im Ergebnis, so dass sekundäre kinetische Isotopeneffekte ausgeschlossen werden können, die die Ergebnisse der Umsetzungen verfälschen könnten.

Sodann wurden etwa 1000 *ee*- und Umsatzbestimmungen pro Tag mit Hilfe der Konfiguration in Schema 2 bei Verwendung von Lipasen (Wildtyp und Mutanten) durchgeführt.

### Beispiel 3. Enantioselektive Hydrolyse von meso-1,4-Diacetoxy-2-cyclopenten

Dieses Beispiel bezieht sich auf die Reaktionen einer prochiralen Verbindung, die enantiotope Gruppen trägt (hier Acetoxygruppen).

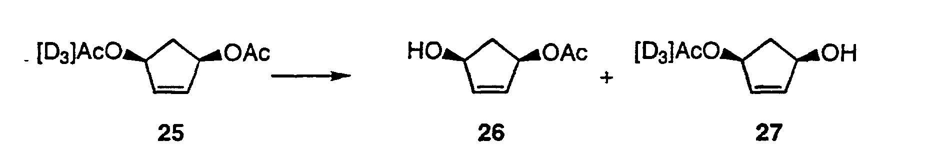

### Synthese von (1S,4R)-cis-1-Acetoxy-4-trideuteroacetoxy-2-cyclopenten (25):

In einem 100 ml-Stickstoffkolben werden unter Luft- und Feuchtigkeitsausschluss 5.79 mmol (823 mg) (1*S*,4*R*)-*cis*-4-Acetoxy-2-cyclopenten-1-ol (Fluka, 99 % *ee*), 6.95 mmol (1.20 Äq., 550 mg, 0.560 ml) Pyridin und 50 ml Dichlormethan vorgelegt und auf 0 °C (Eisbad) gekühlt. Unter Rühren werden innerhalb von 10 min 6.36 mmol (1.10 Äq., 688 mg, 600 µl) -*d*₆-Acetanhydrid (99 Atom% D, Aldrich) zugetropft. Innerhalb von 12 h wird auf Raumtemperatur erwärmt und sukzessive mit je zweimal 40 ml 1 M Salzsäurelösung, gesättigter Natriumhydrogencarbonatlösung und gesättigter Natriumchloridlösung extrahiert. Die organische Phase wird über Magnesiumsulfat getrocknet, vom Trockenmittel durch Filtration getrennt und am Rotationsverdampfer vom Lösungsmittel befreit. Das Rohprodukt wurde auf Kieselgel aufgetragen und chromatographisch gereinigt (Laufmittel: Hexan : Essigsäureethylester = 3 : 1). Die Produktfraktionen werden vereinigt und am Rotationsverdampfer vom Lösungsmittel befreit. Nach Trocknen im Ölpumpenvakuum verbleibt eine klare Flüssigkeit (1.01 g, 5.40 mmol, 93 %). Analytik: ¹H-NMR (CDCl₃, 300 MHz): δ = 1.71-1.78 (m) [2H], 2.07 (s) [3H], 2.83-2.93 (m) [2H], 5.55 (dd) ³*J* = 3.8 Hz, ²*J* = 7.5 Hz [2H], 6.10 (s) [2H]; ¹³C-{¹H}-NMR (CDCl₃, 75 MHz): δ = 21.5 (s), 37.5 (s), 76.9 (s), 135.0 (s), 171.1 (s); MS (EI, 70 eV, pos. Ionen): *m*/*z* (% rel. Int.): 128 (9), 127 (3), 125 (9), 124 (3), 84 (17), 83 (81), 82 (82), 81 (16), 55 (6), 54 (24), 46 (100), 43 (92); EA: C: 57.75 % (ber. 57.74 %), H+D: 6.52 % (ber. 6.46 %).

In Vorversuchen wurde dann die Hydrolyse des Substrats **25** mit einer nichtaktiven Lipase-Mutante (*P. aeruginosa* Mutante) und eines aktiven Enzyms (Schweineleberesterase, Sigma) untersucht. Repräsentative Massenspektren sind in Abbildung 3a und 3b gezeigt.

Die Daten in Abbildung 3a bestätigen das Ausbleiben der Reaktion, während die Daten in Abbildung 3b zu einem *ee*-Wert von 73 % führen. Die unabhängige Analyse mit herkömmlicher Gaschromatographie ergab einen *ee*-Wert 73 %, in exzellenter Übereinstimmung mit der ESI-MS-Analyse. Sodann wurden 1100 Proben pro Tag auf *ee*-Werte hin analysiert.

### Beispiel 4. Enantioselektive Hydrolyse von Bisnaphthylacetaten

In diesem Beispiel wird die enantioselektive Hydrolyse einer 1 : 1-Mischung der *pseudo*-Enantiomere **28** und **29** untersucht.

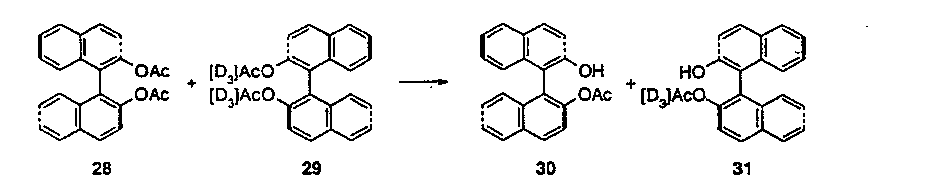

Dazu wurden die *pseudo*-Enantiomere **28** und **29** in enantiomerenreiner Form hergestellt.

### Synthese von (S,S)-1,1'-Bisnaphtyl-2,2'-diacetat (28):

In einem 50 ml-Stickstoffkolben werden unter Luft- und Feuchtigkeitsausschluss 5.00 mmol (1.43 g) (*S*,*S*)-1,1'-Bis-2-naphtol (> 99.9 % *ee*) in 20 ml Dichlormethan gelöst, mit 15.0 mmol (3.00 Äq., 1.19 g, 1.21 ml) Pyridin versetzt und auf 0°C (Eisbad) gekühlt. Unter Rühren werden innerhalb von 5 min 12.5 mmol (2.50 Äq., 985 mg, 888 µl) Acetylchlorid zugetropft. In einem Zeitraum von 12 h wird auf Raumtemperatur erwärmt und anschließend sukzessive mit je zweimal 20 ml 1 M Salzsäure, gesättigter Natriumhydrogencarbonatlösung und gesättigter Natriumchloridlösung extrahiert. Die organische Phase wird über Magnesiumsulfat getrocknet, vom Trockenmittel durch Filtration getrennt und am Rotationsverdampfer vom Lösungsmittel befreit. Das Rohprodukt wurde auf Kieselgel aufgetragen und chromatographisch gereinigt (Laufmittel: Hexan : Essigsäureethylester = 7 : 3). Die Produktfraktionen werden-vereinigt und am Rotationverdampfer vom Lösungsmittel befreit. Nach Trocknen im Ölpumpenvakuum verbleibt ein farbloser Feststoff (1.88 g, 4.99 mmol, 99 %). Analytik: ¹H-NMR (*d*₆-DMSO, 300 MHz): δ = 1.83 (s) [2H], 6.94 (d) *J* = 8.3 Hz 2H], 7.29-7.34 (m) [2H], 7.48-7.55 (m) [4H], 8.05-8.15 (m) [4H]; ¹³C-{1H}-NMR (*d*₆-DMSO, 75 MHz): δ = 20.4 (s), 122.4 (s), 122.6 (s), 125.4 (s), 125.7 (s), 126.8 (s), 128.2 (s), 129.5 (s), 131.0 (s), 132.7 (s), 146.6 (s), 168.8 (s); MS (EI, 70 eV, pos. Ionen): *m*/*z* (% rel. Int.): 370 (13) [M⁺], 328 (37), 286 (100), 268 (9), 257 (7), 239 (10), 46 (11); IR (KBr): 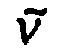 (cm⁻¹) = 3055 (w), 3019 (w). 2936 (w), 1760 (s), 1622 (m), 1595 (m), 1508 (m), 1472 (m), 1430 (m), 1367 (s), 1215 (s), 1130 (s), 1074 (m), 1012 (m), 813 (m), 761 (m); EA: C: 77.53 % (ber. 77.82 %), H: 4.92 % (ber. 4.90 %).

### Synthese von (R,R)-1,1'-Bisnaphtyl-2,2'-bis(trideuteroacetat) (29):

In einem 50 ml-Stickstoffkolben werden unter Luft- und Feuchtigkeitsausschluss 5.00 mmol (1.43 g) (*R*,*R*)-1,1'-Bis-2-naphtol (> 99.9 % *ee*) in 20 ml Dichlormethan gelöst, mit 15.0 mmol (3.00 Äq., 1.19 g, 1.21 ml) Pyridin versetzt und auf 0 °C (Eisbad) gekühlt. Unter Rühren werden innerhalb von 5 min 12.5 mmol (2.50 Äq., 1.019 g, 889 µl) *d*₃-Acetylchlorid (> 99 Atom% D, Aldrich) zugetropft. In einem Zeitraum von 12 h wird auf Raumtemperatur erwärmt und anschließend sukzessive mit je zweimal 20 ml 1 M Salzsäure, gesättigter Natriumhydrogencarbonatlösung und gesättigter Natriumchloridlösung extrahiert. Die organische Phase wird über Magnesiumsulfat getrocknet, vom Trockenmittel durch Filtration getrennt und am Rotationsverdampfer vom Lösungsmittel befreit. Das Rohprodukt wurde auf Kieselgel aufgetragen und chromatographisch gereinigt (Laufmittel: Hexan : Essigsäureethylester = 7 : 3). Die Produktfraktionen werden vereinigt und am Rotationverdampfer vom Lösungsmittel befreit. Nach Trocknen im Ölpumpenvakuum verbleibt ein farbloser Feststoff (1.85 g, 4.99 mmol, 99 %). Analytik: ¹H-NMR (*d*₆-DMSO, 300 MHz): *δ =* 6.94 (d) *J* = 8.3 Hz, 7.27-7.34 (m) [2H], 7.48-7.55 (m) [4H], 8.05-8.15 (m) [4H]; ¹³C-{1H}-NMR (*d*₆-DMSO, 75 MHz): δ = 20.4 (s), 122.4 (s), 122.6 (s), 125.4 (s), 125.7 (s), 126.8 (s), 128.2 (s), 129.5 (s), 131.0 (s), 132.7 (s), 146.6 (s), 168.8 (s); MS (EI, 70 eV, pos. Ionen): *m*/*z* (% rel. Int.): 376 (17) [M⁺], 332 (41), 288 (100), 268 (6), 259 (5), 240 (5), 46 (11); IR (KBr): 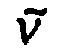 (cm⁻¹) = 3054 (w), 3019 (w), 2268 (w), 2147 (w), 2092 (w), 1755 (s), 1622 (m), 1595 (m), 1509 (m), 1471 (m), 1231 (s), 1146 (s), 1075 (s), 1059 (s), 819 (s), 804 (s), 755 (s); EA: C: 76.23 % (ber. 76.85 %), H+D: 4.89 % (ber. 4.82 %).

Im Rahmen eines *High-Throughput-Screenings* zu *ee-* und Umsatzbestimmungen wurde die in Schema 3 beschriebene Geräteaufbau verwendet.

Wiederum wurden 1 : 1-Mischungen der *pseudo*-Enantiomere hergestellt und im *High-Throughput-Screening* von Lipase-katalysierten kinetischen Racematspaltungen eingesetzt.
Hierzu wurde ein Reflex II MALDI-TOF MS (engl. matrix-assisted laser desorption/ionization time-of-flight mass spectrometer) der Fa. Bruker-Franzen Analytik GmbH, Bremen, genutzt. Das Gerät ist mit einem N₂-Laser (264 nm) und einem 1 GHz *Digitizer* ausgerüstet. Es wurden maximal 40 Spektren automatisch aufgenommen und summiert.
Jede Probe wurde mittels eines Probenvorbereitungsroboters 1 : 1 (ν/ν) mit Matrixlösung (5%ige Trihydroxyacetophenon-Lösung in CH₃OH) versetzt und auf das MALDI-*Target* pipettiert.

In Abbildung 4 sind Ausschnitte ausgewählter MALDI-Massenspektren von Umsetzungen der *pseudo*-Enantiomere **28** und **29** mit Schweineleberesterase (Sigma) gezeigt. Aus den Signalintensitäten [**28** + Na]⁺ und [**29** + Na]⁺ wurden die Enantiomerenüberschüsse bestimmt (Abb. 4a. 40 % *ee,* Abb. 4b 54 % *ee*).

## Patentansprüche

1. Verfahren zur Bestimmung der Enantioselektivität von kinetischen Racematspaltungen an chiralen Substraten oder von stereoselektiven Reaktionen mit enantiotope Gruppen enthaltenden prochiralen Substraten, **dadurch gekennzeichnet, dass**
a) Substrate eingesetzt werden, die eine von der natürlichen Verteilung abweichende Isotopenverteilung aufweisen oder teilweise oder vollständig isotopenmarkiert sind und
b) mit Hilfe einer massenspektrometrischen Ionisierungsmethode der Umsatz oder die relativen Verhältnisse der *pseudo*-Enantiomere oder die Enantiomerenüberschüsse quantitativ bestimmt werden.

2. Verfahren nach Anspruch 1, wobei die quantitative Bestimmung für eine Serie von Proben mit Hilfe eines Probenaufgaberoboters durchgeführt wird.

3. Verfahren nach Anspruch 2, wobei die Proben von einem oder mehreren Probeaufgaberobotem aus einer oder mehreren Mikrotiterplatten in ein oder mehrere Massenspektrometer transferiert werden.

4. Verfahren nach Anspruch 1 - 3, wobei als massenspektrometrische Ionisierungsmethode APCI (atmospheric pressure chemical ionization), CI (chemical ionization), EI (electron ionization), ESI (electrospray ionization), FAB (fast atom bombardment), FD (field desorption), FI (field ionization), LILBID (laser induced liquid beam ionization desorption), LSIMS (liquid secondary ion mass spectrometry), MALDI (matrix-assisted laser desorption/ionization), PB (particle beam), PD (plasma desorption), SIMS (secondary ion mass spectrometry), oder TSP (thermospray) oder eine Kombination solcher Ionisierungsmethoden angewandt wird.

5. Verwendung des Verfahrens gemäß Ansprüchen 1 - 4
a) zur Auffindung oder Optimierung von Katalysatoren, die Reaktionen asymmetrisch katalysieren oder
b) zur Auffindung von chiralen Agenzien, die in Reaktionen zur Anreicherung eines Enantiomers führen.

6. Verwendung nach Anspruch 5a) zur Auffindung von Katalysatoren für die kinetische Racematspaltung von Alkoholen, Carbonsäuren, Carbonsäureestern, Aminen, Amiden, Olefinen, Alkinen, Phosphinen, Phosphoniten, Phosphiten, Phosphaten, Halogeniden, Oxiranen, Thiolen, Sulfiden, Sulfonen, Sulfoxiden, Sulfonamiden sowie deren Derivate.

7. Verwendung nach Anspruch 5a) zur Auffindung von chiralen Katalysatoren oder Agenzien für die stereoselektive Umsetzung von prochiralen Verbindungen, deren enantiotope Gruppen eine oder mehrere funktionelle Gruppen aus den Substanzklassen der Alkohole, Carbonsäuren, Carbonsäureester, Amine, Amide, Olefine, Alkine, Phosphine, Phosphonite, Phosphite, Phosphate, Halogenide, Oxirane, Thiole, Sulfide, Sulfone, Sulfoxide oder Sulfonamide oder deren Derivate umfassen.

8. Verwendung nach Anspruch 5a) zur Auffindung von Biokatalysatoren für die stereoselektive Synthese.

9. Verwendung nach Anspruch 8, wobei Biokatalysatoren Enzyme, Antikörper, Proteine, Hormone, Phagen, Ribozyme, Peptide oder sonstige Biopolymere sein können.

10. Verwendung nach Anspruch 5a) zur Auffindung von chiralen homogenen Katalysatoren für die stereoselektive Synthese.

11. Verwendung nach Anspruch 5a) zur Auffindung von chiralen Metallkomplexen für die stereoselektive Synthese.

12. Verwendung nach Anspruch 5a) zur Auffindung von chiral modifizierten heterogenen Katalysatoren für die stereoselektive Synthese.

## Claims

1. Method for determining the enantioselectivity of kinetic racemate resolutions on chiral substrates or of stereoselective reactions using prochiral substrates which contain enantiotopic groups, where
a) use is made of substrates which exhibit an isotope distribution which differs from the natural distribution or which are partially or completely isotope-labelled, and
b) a mass spectrometric ionization method is used to quantitatively determine the conversion, or the relative ratio, of the pseudo-enantiomers or the enantiomeric excesses.

2. Method according to Claim 1, where the quantitative determination for a series of samples is carried out using a sampling robot.

3. Method according to Claim 2, where the samples are transferred, by one or more sampling robots, from one or more microtitre plates to one or more mass spectrometers.

4. Method according to Claims 1-3, where the mass spectrometric ionization method employed is APCI (atmospheric pressure chemical ionization), CI (chemical ionization), EI (electron ionization), ESI (electrospray ionization), FAB (fast atom bombardment), FD (field desorption), FI (field ionization), LILBID (laser induced liquid beam ionization desorption), LSIMS (liquid secondary ion mass spectrometry), MALDI (matrix-assisted laser desorption/ionization), PB (particle beam), PD (plasma desorption), SIMS (secondary ion mass spectrometry), or TSP (thermospray), or a combination of these ionization methods.

5. Use of the method according to Claims 1-4
a) for finding or optimizing catalysts which catalyse reactions asymmetrically, or
b) for finding chiral agents which lead, in reactions, to the enrichment of an enantiomer.

6. Use according to Claim 5a) for finding catalysts for the kinetic racemate resolution of alcohols, carboxylic acids, carboxylic acid esters, amines, amides, olefins, alkynes, phosphines, phosphonites, phosphites, phosphates, halides, oxiranes, thiols, sulphides, sulphones, sulphoxides, sulphamides and their derivatives.

7. Use according to Claim 5 for finding chiral catalysts or agents for the stereoselective conversion of prochiral compounds whose enantiotopic groups comprise one or more functional groups from the substance classes of the alcohols, carboxylic acids, carboxylic acid esters, amines, amides, olefins, alkynes, phosphines, phosphonites, phosphites, phosphates, halides, oxiranes, thiols, sulphides, sulphones, sulphoxides or sulphonamides or their derivatives.

8. Use according to Claim 5a) for finding biocatalysts for the stereoselective synthesis.

9. Use according to Claim 8, where biocatalysts can be enzymes, antibodies, proteins, hormones, phages, ribozymes, peptides or other biopolymers.

10. Use according to Claim 5a) for finding homogeneous chiral catalysts for the stereoselective synthesis.

11. Use according to Claim 5a) for finding chiral metal complexes for the stereoselective synthesis.

12. Use according to Claim 5a) for finding chirally modified heterogeneous catalysts for the stereoselective syntheses.

## Revendications

1. Procédé pour la détermination de l'énantiosélectivité de dissociations cinétiques de racémates sur des substrats chiraux ou de réactions stéréosélectives avec des substrats prochiraux contenant des groupements énantiotopes, **caractérisé en ce qu'**on
a) utilise des substrats qui présentent une répartition d'isotopes s'écartant de la répartition naturelle ou qui sont marqués partiellement ou totalement par des isotopes et
b) détermine quantitativement la transformation ou les rapports relatifs des pseudo-énantiomères ou les excès d'énantiomères à l'aide d'un procédé d'ionisation de spectrométrie de masse.

2. Procédé selon la revendication 1, la détermination quantitative étant réalisée pour une série d'échantillons à l'aide d'un robot d'alimentation en échantillons.

3. Procédé selon la revendication 2, les échantillons étant transférés d'une ou de plusieurs plaques de microtitrage dans un ou plusieurs spectromètres de masse par un ou plusieurs robots d'alimentation en échantillons.

4. Procédé selon la revendication 1 à 3, avec utilisation, comme procédé d'ionisation de spectrométrie de masse, des procédés APCI (atmospheric pressure chemical ionization - ionisation chimique à pression atmosphérique), CI (chemical ionization - ionisation chimique), EI (electron ionization - ionisation électronique), ESI (electrospray ionization
- ionisation par électrospray), FAB (fast atom bombardment - bombardement d'atomes rapides), FD (field desorption - désorption de champ), FI (field ionization
- ionisation de champ), LILBID (laser induced liquid beam ionization desorption - ionisation/désorption de faisceau liquide induit par laser), LSIMS (liquid secondary ion mass spectrometry - spectrométrie de masse à ions secondaires avec cible liquide), MALDI (matrix-assisted laser desorption/ionization - désorption/ionisation laser assistée par matrice), PB (particle beam - faisceau de particules), PD (plasma desorption - désorption de plasma), SIMS (secondary ion mass spectrometry - spectrométrie de masse à ions secondaires) ou TSP (thermospray) ou une combinaison de ces procédés d'ionisation.

5. Utilisation du procédé selon les revendications 1 à 4
a) pour mettre au point ou optimiser des catalyseurs qui catalysent asymétriquement des réactions ou
b) pour mettre au point des agents chiraux qui conduisent à l'enrichissement d'un énantiomère dans des réactions.

6. Utilisation selon la revendication 5a) pour mettre au point des catalyseurs pour la dissociation cinétique de racémates d'alcools, d'acides carboxyliques, d'esters d'acide carboxylique, d'amines, d'amides, d'oléfines, d'alcynes, de phosphines, de phosphonites, de phosphites, de phosphates, d'halogénures, d'oxirannes, de thiols, de sulfures, de sulfones, de sulfoxydes, de sulfonamides ainsi que de leurs dérivés.

7. Utilisation selon la revendication 5a) pour mettre au point des catalyseurs ou des agents chiraux pour la transformation stéréosélective de composés prochiraux, dont les groupements énantiotopes comprennent un ou plusieurs groupements fonctionnels des classes de substances des alcools, des acides carboxyliques, des esters d'acide carboxylique, des amines, des amides, des oléfines, des alcynes, des phosphines, des phosphonites, des phosphites, des phosphates, des halogénures, des oxirannes, des thiols, des sulfures, des sulfones, des sulfoxydes ou des sulfonamides ou de leurs dérivés.

8. Utilisation selon la revendication 5a) pour mettre au point des biocatalyseurs pour la synthèse stéréosélective.

9. Utilisation selon la revendication 8, les biocatalyseurs pouvant être des enzymes, des anticorps, des protéines, des hormones, des phages, des ribozymes, des peptides ou d'autres biopolymères.

10. Utilisation selon la revendication 5a) pour mettre au point des catalyseurs homogènes chiraux pour la synthèse stéréosélective.

11. Utilisation selon la revendication 5a) pour mettre au point des complexes métalliques chiraux pour la synthèse stéréosélective.

12. Utilisation selon la revendication 5a) pour mettre au point des catalyseurs hétérogènes modifiés de manière chirale pour la synthèse stéréosélective.
